(19) Europäisches Patentamt
European Patent Office
Office européen des brevets

(11) **EP 4 309 658 A1**

(12) **EUROPEAN PATENT APPLICATION**
published in accordance with Art. 153(4) EPC

(43) Date of publication:
**24.01.2024 Bulletin 2024/04**

(21) Application number: **22766372.1**

(22) Date of filing: **11.03.2022**

(51) International Patent Classification (IPC):
**A61K 31/57** (2006.01)   **A61K 39/395** (2006.01)
**A61P 35/00** (2006.01)   **A61P 35/04** (2006.01)

(52) Cooperative Patent Classification (CPC):
**A61K 31/57; A61K 39/395; A61K 45/06;**
**A61P 35/00; A61P 35/04**

(86) International application number:
**PCT/CN2022/080275**

(87) International publication number:
**WO 2022/188850 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.03.2021   CN 202110265778**

(71) Applicant: **Shenzhen Evergreen Therapeutics Co., Ltd.**
**Shenzhen, Guangdong 518038 (CN)**

(72) Inventors:
• **DU, Tao Tom**
  **Shenzhen, Guangdong 518038 (CN)**
• **HU, Tao**
  **Shenzhen, Guangdong 518038 (CN)**
• **ZHANG, Yuqian**
  **Shenzhen, Guangdong 518038 (CN)**
• **DU, Xin**
  **Shenzhen, Guangdong 518038 (CN)**

(74) Representative: **Groth & Co. KB**
**P.O. Box 6107**
**102 32 Stockholm (SE)**

(54) **ANTI-TUMOR COMBINED PREPARATION COMPRISING HYDROXYPROGESTERONE CAPROATE AND USE THEREOF**

(57)    Provided in the present invention is a pharmaceutical composition comprising hydroxyprogesterone caproate and an additional therapeutic agent for treating tumors, wherein the additional therapeutic agent is preferably a monoclonal antibody against PD-1 or PD-L1.

Further provided in the present invention are a method for increasing the number of tumor infiltrating lymphocytes (TIL) as required and a method for treating cancers in a subject in need thereof, which comprises providing the hydroxyprogesterone caproate to the subject.

Figure 1

**Description**

**Cross Reference to Related Application**

The present application claims the priority of the Chinese patent application filed with the China National Intellectual Property Administration on March 11, 2021, with application number 202110265778.5, titled "Anti-Tumor Combination Preparation Containing Hydroxyprogesterone Acetate and Its Applications." The entire content thereof is incorporated herein by reference in this application.

**Technical Filed**

The present invention relates to a pharmaceutical composition for treating tumors and a method for treating tumors, particularly involving a pharmaceutical composition that enhances the effectiveness of tumor therapeutics by improving the tumor microenvironment and a method for treating tumors through combination therapy.

**Background**

Malignant tumors (cancer) pose a significant threat to human health. Conventional treatment methods include surgery, radiation therapy, chemotherapy, and targeted therapy. However, their effectiveness against advanced-stage tumors is limited and often accompanied by substantial toxicity and poor tolerability issues. In recent years, tumor immunotherapy has shown remarkable clinical efficacy. It is a treatment modality that activates the body's own immune system to combat tumors and is considered the most promising approach for curing malignant tumors. As such, tumor immunotherapy was honored with the Nobel Prize in Physiology or Medicine in 2018.

The tumor microenvironment is a crucial aspect of cancer biology, contributing to tumor initiation, progression, and responses to treatment. Cells and molecules of the immune system constitute fundamental components of the tumor microenvironment. The composition and characteristics of the tumor microenvironment vary significantly and play a vital role in determining the anti-tumor immune response. For instance, certain immune system cells, including natural killer cells, dendritic cells (DCs), and effector T cells, can drive robust anti-tumor responses. However, tumor cells often induce an immunosuppressive microenvironment, favoring the development of immunosuppressive populations of immune cells, such as myeloid-derived suppressor cells and regulatory T cells.

Immune checkpoint inhibitors, as representatives of tumor immunotherapy, function by blocking immunosuppressive signals in the tumor microenvironment, restoring the functionality of immune cells to exert anti-tumor effects. Among these, the most extensively researched and clinically applied immune checkpoints include programmed cell death protein 1 (PD-1), programmed death-ligand 1 and 2 (PD-L1/L2), cytotoxic T-lymphocyte-associated protein 4 (CTLA-4), lymphocyte activation gene 3 (LAG-3), T-cell immunoglobulin and mucin domain-containing protein 3 (TIM-3), T-cell immunoglobulin and ITIM domain (TIGIT), and V-domain Ig suppressor of T-cell activation (VISTA) (Randolph J. Nolle, Science, 2020).

In the tumor microenvironment, PD-1 and/or PD-L1 are highly expressed in most tumor-infiltrating lymphocytes (TILs) from various tumor types. They inhibit localized immune responses, including lung cancer, liver cancer, ovarian cancer, cervical cancer, skin cancer, colon cancer, glioma, bladder cancer, breast cancer, kidney cancer, esophageal cancer, stomach cancer, oral squamous cell carcinoma, urothelial carcinoma, pancreatic cancer, and head and neck tumors. PD-1 and/or PD-L1 antibodies can block the binding of PD-L1 on tumor cells with the inhibitory receptor PD-1 on the surface of activated T cells, thereby activating tumor-specific T cells and restoring the immune system's ability to eliminate tumor cells. However, due to the substantial heterogeneity and genetic instability of tumors, their complex pathogenesis leads to various endogenous and exogenous immune resistance mechanisms. PD-1 antibodies exhibit a clinical response rate of only about 20%.

The inventors discovered through research that not only PD-1 antibodies but also other antibodies and various tumor therapeutics show significant differences in clinical efficacy compared to in vitro experiments. An important reason for this discrepancy is the tumor's microenvironment, which is conducive to cancer cell survival and proliferation. This results in challenges such as drugs struggling to reach their targets and immune cells finding it difficult to penetrate tumor tissues to eliminate cancer cells.

17$\alpha$-Hydroxyprogesterone caproate, also known as hydroxyprogesterone caproate, 17$\alpha$-hydroxyprogesterone acetate, 17-OHPC, OHPC, with the chemical formula $C_{27}H_{40}O_4$ and CAS No. 630-56-8, is clinically used for treating habitual abortion, menstrual disorders, endometriosis, functional uterine bleeding, etc.

**Summary of The Invention**

According to one aspect of the present invention, a pharmaceutical composition is provided, comprising hydroxyprogesterone caproate and an additional therapeutic agent for treating tumors.

Optionally, the additional therapeutic agent for treating tumors is selected from: chemotherapeutic agents, targeted therapeutic agents, hormonal therapeutic agents, cellular therapeutic agents, oncolytic viral agents, or antibodies, particularly immune checkpoint inhibitors.

Optionally, the immune checkpoint inhibitor is selected from inhibitors of PD-L1, PD-1, PD-L2, CTLA-4, LAG3, B7-H3, KIR, CD137, PS, TFM3, CD52, CD30, CD20, CD33, CD27, OX40, GITR, ICOS, BTLA (CD272), CD160, 2B4, LAIR1, TIGHT, LIGHT, DR3, CD226, CD2, SLAM, or combinations thereof. Preferably, the immune checkpoint inhibitor is a monoclonal antibody, particularly targeting PD-1 or PD-L1.

Optionally, the additional therapeutic agent is selected from atezolizumab, avelumab, durvalumab, ceralasertib, cemiplimab, ipilimumab, nivolumab, pembrolizumab, sintilimab, tremelimumab, tislelizumab, toripalimab, tiragolumab, or RC98.

According to another aspect of the invention, an anti-tumor drug is provided, comprising the aforementioned pharmaceutical composition containing hydroxyprogesterone caproate and another therapeutic agent for treating tumors, and optionally one or more pharmaceutically acceptable excipients.

Optionally, the formulation of the anti-tumor drug is selected from injectable, oral, or topical formulations; preferably, an injectable formulation; particularly preferably, an injectable liquid or powder for injection.

Optionally, the content of the additional therapeutic agent is 10%-100% of the effective dose when used alone. For instance, in a daily tablet/dose formulation, the content of hydroxyprogesterone caproate is 100-1000mg per tablet/dose; for a formulation with m tablets/doses used daily, the content of hydroxyprogesterone caproate is (100-1000mg)/m per tablet/dose; for a formulation used every n days, the content of hydroxyprogesterone caproate is $n \times$ (100-1000mg) per tablet/dose.

Optionally, the pharmaceutically acceptable excipients include solvents, cosolvents, solubilizers, emulsifiers, colorants, binders, disintegrants, fillers, lubricants, wetting agents, osmotic pressure regulators, stabilizers, flow aids, masking agents, preservatives, suspending agents, coating materials, fragrances, anti-adherents, integrators, permeation enhancers, pH regulators, buffers, plasticizers, surfactants, thickeners, encapsulating agents, moisturizers, absorbents, diluents, flocculating agents, anti-flocculating agents, aid filters, release retardants, high molecular weight skeleton materials, and film-forming materials, at least one of which is used.

According to another aspect of the invention, a method is provided for increasing the number of tumor-infiltrating lymphocytes (TILs) in a subject when needed, comprising providing hydroxyprogesterone caproate to the subject.

Optionally, the TIL population comprises CD8+ T cells, CD4+ T cells, B cells, natural killer (NK) cells, dendritic cells (DCs), M1 macrophages, or combinations thereof, preferably expressing the TH1 phenotype of T cells.

Optionally, the increase in TIL number occurs in the tumor microenvironment (TME) and/or within the tumor tissue.

According to another aspect of the invention, a method is provided for treating cancer in a subject in need, comprising providing hydroxyprogesterone caproate to the subj ect.

Optionally, the method includes providing an additional therapeutic agent for treating cancer, apart from hydroxyprogesterone caproate.

Optionally, the additional therapeutic agent is selected from chemotherapeutic agents, targeted therapeutic agents, hormonal therapeutic agents, cellular therapeutic agents, oncolytic viral agents, or antibodies.

Optionally, the additional therapeutic agent is selected from immune therapeutic agents.

Optionally, the additional therapeutic agent is selected from immune checkpoint inhibitors.

Optionally, the immune checkpoint inhibitor is selected from inhibitors of PD-L1, PD-1, PD-L2, CTLA-4, LAG3, B7-H3, KIR, CD137, PS, TFM3, CD52, CD30, CD20, CD33, CD27, OX40, GITR, ICOS, BTLA (CD272), CD160, 2B4, LAIR1, TIGHT, LIGHT, DR3, CD226, CD2, SLAM, preferably targeting PD-1, PD-L2, CTLA-4, LAG3, B7-H3, KIR, CD137, PS, TFM3, CD52, CD30, CD20, CD33, CD27, OX40, GITR, ICOS, BTLA (CD272), CD160, 2B4, LAIR1, TIGHT, LIGHT, DR3, CD226, CD2, SLAM, or combinations thereof, with monoclonal antibodies particularly targeting PD-L1 or PD-1.

Optionally, the additional therapeutic agent is selected from atezolizumab, avelumab, durvalumab, ceralasertib, cemiplimab, ipilimumab, nivolumab, pembrolizumab, sintilimab, tremelimumab, tislelizumab, toripalimab, tiragolumab, or RC98.

Optionally, the subject has high PD-L1 or PD-1 expression.

Optionally, it is applied as a first-line or second-line treatment method.

Optionally, hydroxyprogesterone caproate can be administered to the subject before, simultaneously, or after administering the additional therapeutic agent.

Optionally, the subject suffers from primary or metastatic cancer. The cancer includes solid tumors or metastatic cancers, where solid tumors are bladder cancer, bone cancer, brain cancer, breast cancer, colorectal cancer, endometrial cancer, esophageal cancer, eye cancer, head and neck cancer, kidney cancer, lung cancer, melanoma, ovarian cancer, pancreatic cancer, prostate cancer, or gastric cancer. Preferably, breast cancer, lung cancer, renal cell carcinoma (RCC), hepatocellular carcinoma (HCC), melanoma, colorectal cancer, or endometrial cancer, particularly primary or metastatic non-small cell lung cancer (NSCLC), and the metastatic cancer includes metastatic melanoma, metastatic colorectal cancer, or metastatic endometrial cancer.

Optionally, the subject suffers from recurrent cancer or refractory cancer.

Optionally, using hydroxyprogesterone caproate or using hydroxyprogesterone caproate with an additional therapeutic agent for treating cancer reduces tumor cell proliferation in the subject and/or slows down or stabilizes tumor growth.

**Brief Description of The Drawings**

To provide a clearer explanation of specific embodiments of the invention or technical solutions in the prior art, a brief introduction to the drawings required for the description of specific embodiments or prior art will be provided below. It is evident that the illustrations in the description below represent certain embodiments of the present invention. Those skilled in the art can derive other illustrations based on these illustrations without exercising inventive labor.

Figure 1 depicts the trend of tumor volume growth after administration.

Figure 2 illustrates the infiltration of CD8 T cells in mouse tumors post-administration (400X magnification).

Figure 3 presents the analysis of CD8 staining density on tumor sections after administration, represented in integrated optical density (IOD)/area, where ** denotes $p < 0.01$ compared between the OHPC group and the control group.

**Detailed Description**

The provided embodiments are meant to enhance the understanding of the present invention and are not limited to the described best embodiments. They do not restrict the content and scope of the invention. Any product obtained by anyone under the guidance of the present invention or by combining the features of the present invention with other features of the prior art falls within the protection scope of the present invention.

If specific experimental steps or conditions are not indicated in the embodiments, the operations or conditions can be carried out according to the conventional experimental procedures described in the literature within this field. Reagents or instruments not specified by manufacturers are standard products available for purchase in the market.

**Example 1**

1. Investigational Drugs and Reagents

Antibody Anti-PD-L1 was purchased from Rongchang Biopharmaceuticals (Yantai) Co., Ltd., batch number: RC98-1-A20170426, with a storage solution concentration of 6.87 mg/ml, dissolved in 0.9% sodium chloride solution. Prior to each administration, the Anti-PD-L1 was prepared by diluting it in 0.9% sodium chloride solution to obtain a concentration of 1 mg/ml.

Hydroxyprogesterone Caproate (OHPC) was purchased from Hongyan Pharmaceutical and Chemical Co., Ltd., as a powder. Prior to each administration, OHPC powder was dissolved in DMSO. Once fully dissolved, it was diluted in sesame oil to obtain an OHPC concentration of 4 mg/ml for the investigational drug solution.

2. Animal Grouping, Modeling, and Drug Administration

(1) Cell Culture

Murine breast cancer 4T1 cells were purchased from Beijing Biosynthgene Biotech Co., Ltd. Cells were cultured at 37°C and 5% $CO_2$ in DMEM culture medium containing 10% inactivated fetal bovine serum. The 4T1 cells were genetically modified using conventional methods to overexpress human PD-L1, and these cells were named 4T1-hPD-1.

(2) Tumor Cell Inoculation, Grouping, and Drug Administration

Female Balb/c mice (6-9 weeks old, weighing 16-22g), SPF grade, were subcutaneously inoculated with $5 \times 10^4$ cells/0.1ml concentration of 4T1-hPD-1 cells suspended in PBS at a volume of 0.1ml on the right side. When the average tumor volume reached approximately 100mm3, appropriate mice were selected based on tumor volume and weight and were evenly distributed into four experimental groups, each with 8 mice. Drug administration commenced on the same day as outlined in the table below:

Table 1 Drug Administration

| Group | Test Product, Dose (mg/kg)[a] | Animal Number | Frequency | Number of Administration |
|---|---|---|---|---|
| Control Group (G1) | PBS (Intramuscular) | 8 | Twice per week | 6 |
| Anti-PD-L1(G2) | Anti-PD-L1 (10mg/kg, Intravenous) | 8 | Twice per week | 6 |
| OHPC(G3) | OHPC (20mg/kg, Intramuscular) | 8 | Twice per week | 6 |
| Anti-PD-L1+OHPC(G4) | Anti-PD-L1 (10mg/kg, Intravenous) + OHPC (20mg/kg, Intramuscular) | 8 | Twice per week | 6 |
| a: The dosage volume was calculated at 10μl/g of the experimental animal's volume. | | | | |

After the final administration, the body weight and tumor growth status of the experimental animals were observed for an additional 12 days. Throughout the observation period, tumor volume and animal weight were measured weekly, and the values were recorded. Upon completion of the observation period, the experiment was concluded.

3. Measurement Parameters

(1) Tumor Volume

Measurements of tumor volume were conducted twice a week post-grouping using a vernier caliper. Prior to euthanasia, tumor dimensions including the longest and shortest diameters were measured. The formula used to calculate tumor volume: Tumor Volume = $0.5 \times$ Longest Diameter $\times$ Shortest Diameter$^2$.

(2) Body Weight Monitoring

Animals were weighed at various intervals: upon inoculation, pre-dosing (i.e., before the initial administration), twice a week during drug administration, and before euthanasia.

(3) General Clinical Observations

Daily observations were carried out throughout the adaptation period and the experimental phase. Observations included but were not limited to monitoring tumor nodules for ulceration, assessing the animals' behavioral and dietary patterns.

4. Evaluation Criteria for Drugs

(1) Tumor Growth Inhibition Rate (TGITV)

$$TGI_{TV} (\%) = [1 - (Ti - T0) / (Vi - V0)] \times 100\%;$$

Ti represents the mean tumor volume of the treatment group on day i of drug administration, T0 denotes the mean tumor volume of the treatment group on day 0 of drug administration. Vi stands for the mean tumor volume of the solvent control group on day i of drug administration, and V0 indicates the mean tumor volume of the solvent control group on day 0 of drug administration.

(2) Infiltration of CD8+ T Cells in Tumor Tissue

At the conclusion of the experiment, tumor tissue was embedded in paraffin, sectioned, and stained for CD markers. This was conducted to compare the infiltration of CD8+ T cells within tumors across different groups.

5. Data Collection and Statistical Analysis

Original data from observations and measurements were recorded. Analysis and processing were based on this raw data. Results were expressed as Mean $\pm$ Standard Error of Mean (SEM). Statistical analysis was performed on tumor volume data, where $P < 0.05$ was considered statistically significant. Analysis was conducted considering both statistical and biological significance.

6. Experimental Results

(1) Tumor Volume Inhibition Results

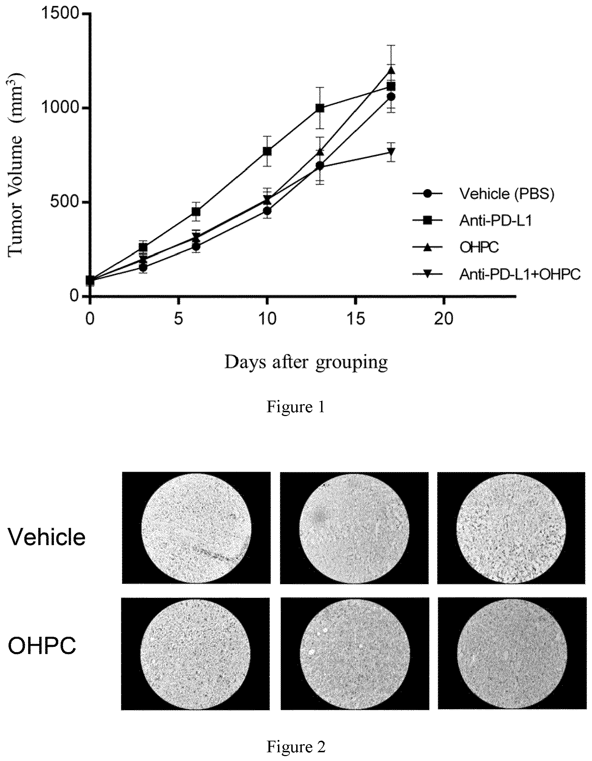

Figure 1 shows the trend of tumor volume growth post-administration. As depicted in Figure 1, 4T1 breast cancer demonstrated resistance to immunotherapy, hence the anti-PD-L1 group didn't exhibit any significant tumor growth inhibition. Similarly, the administration of OHPC alone showed no inhibitory effect. However, in the combination group of anti-PD-L1 and OHPC, there was a noticeable suppression of tumor growth in the later stages. By the 17th day, the experiment's conclusion, the inhibition rate reached 30.7%.

Results for tumor volume and tumor volume inhibition rates for each group are listed in Table 2.

Table 2 Effect of Test Products on Tumor Volume in Balb/c Mice Injected with 4T1-hPD-L1 Cells

| Group | Test Product | Tumor Volume ($mm^3$)[a] | | | $P$[b] |
|---|---|---|---|---|---|
| | | Pre-administration | Day 17 | $TGI_{TV}$(%) | |
| G1 | Vehicle(PBS) | 84±27 | 1062±245 | - | - |
| G2 | Anti-PD-L1 | 89±18 | 1116±234 | -4.9 | 0.725 |
| G3 | OHPC | 87±24 | 1203±384 | -14 | 0.398 |
| G4 | Anti-PD-L1+OHPC | 88±18 | 766±81 | 30.7 | 0.016 |
| Note: a: Mean $\pm$ Standard Error b: Comparison of group weight to Vehicle control weight on the 17th day of dosing was analyzed using t-test. | | | | | |

(2) Infiltration of CD8+ T Cells in Tumor Tissue

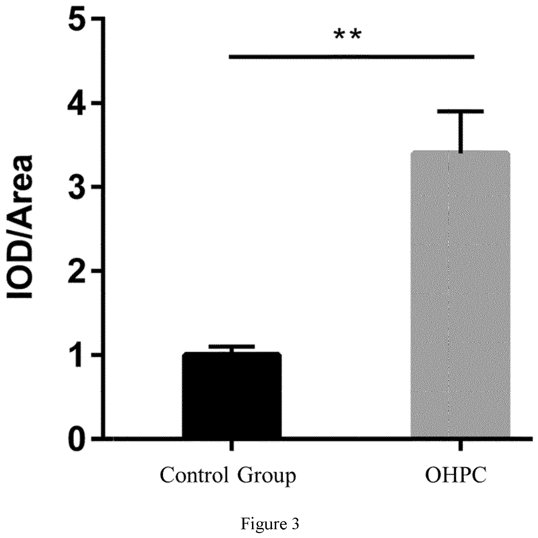

Refer to Supplementary Figures 2 and 3 in the manual; Hydroxyprogesterone Caproate (OHPC) notably enhanced the infiltration of CD8+ T cells.

7. Result

The hydroxyprogesterone caproate significantly enhances the infiltration of CD8+ T cells and augments the anti-tumor activity of the monoclonal antibody Anti-PD-L1.

Evidently, the embodiments are presented solely for illustrative purposes and do not limit the implementation. Ordinary skilled practitioners in this field may introduce other modifications or variations based on the provided descriptions. It is not necessary nor feasible to exhaustively list all possible embodiments. The evident variations or alterations derived from this remain within the scope of protection provided by this invention.

**Claims**

1. A pharmaceutical composition comprising hydroxyprogesterone acetate and an additional therapeutic agent for treating tumors.

2. The pharmaceutical composition of claim 1, wherein the additional therapeutic agent for treating tumors is selected from: chemotherapeutic agents, targeted therapy agents, hormonal therapy agents, cell therapy agents, oncolytic viral agents, or antibodies.

3. The pharmaceutical composition of claim 1 or 2, wherein the additional therapeutic agent is an immune checkpoint inhibitor.

4. The pharmaceutical composition of claim 3, wherein the immune checkpoint inhibitor targets PD-L1, PD-1, PD-L2, CTLA-4, LAG3, B7-H3, KIR, CD137, PS, TFM3, CD52, CD30, CD20, CD33, CD27, OX40, GITR, ICOS, BTLA (CD272), CD160, 2B4, LAIR1, TIGHT, LIGHT, DR3, CD226, CD2, or SLAM, preferably a monoclonal antibody targeting PD-1 or PD-L1.

5. The pharmaceutical composition of claim 4, wherein the additional therapeutic agent is selected from atezolizumab, avelumab, durvalumab, cemiplimab, siltuximab, ipilimumab, nivolumab, pembrolizumab, sintilimab, tislelizumab, tremelimumab, tisotumab vedotin, or RC98.

6. An anti-tumor drug comprising any one of the pharmaceutical compositions of claims 1 to 5 and optionally one or more pharmaceutically acceptable excipients.

7. The anti-tumor drug of claim 6, wherein the drug formulation is selected from injectable formulations, oral formulations, or topical formulations; preferably injectable or oral formulations; particularly preferably injectable formulations.

8. The anti-tumor drug of claim 7, wherein the injectable formulation is selected from injectable solutions or powder for injection; the oral formulation is selected from tablets, solutions, capsules, powders, pills, granules, syrups, suspensions, or orally disintegrating controlled-release formulations; the topical formulation is selected from ointments, sprays, or patches.

9. The anti-tumor drug of any one of claims 6 to 8, wherein the content of the additional therapeutic agent is 10%-100% of the effective dose for individual use.

10. The anti-tumor drug of any one of claims 6 to 9, wherein in a once-a-day tablet/dose formulation, the content of hydroxyprogesterone acetate per tablet/dose is 100-1000mg; in a formulation used m times daily, the content of hydroxyprogesterone acetate per tablet/dose is (100-1000mg)/m; in a formulation used once every n days, the content of hydroxyprogesterone acetate per tablet/dose is $n \times (100\text{-}1000)$mg.

11. A method of increasing the number of tumor-infiltrating lymphocytes (TIL) when needed, comprising administering hydroxyprogesterone acetate to a subject.

12. The method of claim 11, wherein the TIL population comprises a group of CD8+ T cells, CD4+ T cells, B cells, natural killer (NK) cells, dendritic cells (DCs), M1 macrophages, or a combination thereof, preferably expressing a TH1 phenotype of T cells.

13. A method as claimed in claims 11 or 12, wherein the increase in TIL numbers occurs in the tumor microenvironment (TME) and/or within tumor tissues.

14. A method for treating cancer in a subject in need thereof, comprising administering hydroxyprogesterone acetate to the subject.

15. A method as claimed in any one of claims 11 to 14, further comprising administering an additional therapeutic agent for treating cancer to the subject, apart from hydroxyprogesterone acetate.

16. A method as claimed in claim 15, wherein the additional therapeutic agent is selected from chemotherapeutic agents, targeted therapy agents, hormonal therapy agents, cell therapy agents, oncolytic viral agents, or antibodies.

17. A method as claimed in claim 15, wherein the additional therapeutic agent is an immune therapy agent.

18. A method as claimed in claim 17, wherein the additional therapeutic agent is an immune checkpoint inhibitor.

19. A method as claimed in claim 18, wherein the immune checkpoint inhibitor is selected from inhibitors of PD-L1, PD-1, PD-L2, CTLA-4, LAG3, B7-H3, KIR, CD137, PS, TFM3, CD52, CD30, CD20, CD33, CD27, OX40, GITR, ICOS, BTLA (CD272), CD160, 2B4, LAIR1, TIGHT, LIGHT, DR3, CD226, CD2, SLAM, or a monoclonal antibody targeting PD-1, PD-L2, CTLA-4, LAG3, B7-H3, KIR, CD137, PS, TFM3, CD52, CD30, CD20, CD33, CD27, OX40, GITR, ICOS, BTLA(CD272), CD160, 2B4, LAIR1, TIGHT, LIGHT, DR3, CD226, CD2, SLAM, or a combination thereof, particularly preferably a monoclonal antibody targeting PD-L1 or PD-1.

20. A method as claimed in any one of claims 15 to 19, wherein the additional therapeutic agent is selected from atezolizumab, avelumab, durvalumab, cemiplimab, siltuximab, ipilimumab, nivolumab, pembrolizumab, sintilimab, tislelizumab, tremelimumab, tisotumab vedotin, or RC98.

21. A method as claimed in any one of claims 15 to 20, wherein the subject has higher expression levels of PD-L1 or PD-1.

22. A method as claimed in any one of claims 11 to 21, used as a first-line or second-line treatment method.

23. A method as claimed in any one of claims 15 to 22, wherein hydroxyprogesterone acetate is administered to the subject before, simultaneously with, or after administering the additional therapeutic agent.

24. A method as claimed in any one of claims 11 to 23, wherein the subject has primary or metastatic cancer.

25. A method as claimed in claim 24, wherein the cancer is a solid tumor or metastatic cancer, wherein the solid tumor is bladder cancer, bone cancer, brain cancer, breast cancer, colorectal cancer, endometrial cancer, esophageal cancer, eye cancer, head and neck cancer, kidney cancer, lung cancer, melanoma, ovarian cancer, pancreatic cancer, prostate cancer, or gastric cancer, preferably breast cancer, lung cancer, renal cell carcinoma (RCC), hepatocellular carcinoma (HCC), melanoma, colorectal cancer, or endometrial cancer, particularly preferably primary or metastatic non-small cell lung cancer (NSCLC), and the metastatic cancer is metastatic melanoma, metastatic colorectal cancer, or metastatic endometrial cancer.

26. A method as claimed in any one of claims 14 to 25, wherein the subject has recurrent cancer or refractory cancer.

27. A method as claimed in any one of claims 14 to 26, wherein the use of hydroxyprogesterone acetate or the use of hydroxyprogesterone acetate in combination with an additional therapeutic agent for treating cancer reduces tumor cell proliferation in the subject and/or slows down or stabilizes tumor growth in the subject.

Figure 1

Figure 2

Figure 3

## INTERNATIONAL SEARCH REPORT

| International application No. |
| --- |
| **PCT/CN2022/080275** |

**A. CLASSIFICATION OF SUBJECT MATTER**

A61K 31/57(2006.01)i; A61K 39/395(2006.01)i; A61P 35/00(2006.01)i; A61P 35/04(2006.01)i

According to International Patent Classification (IPC) or to both national classification and IPC

**B. FIELDS SEARCHED**

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

CNABS; DWPI; VEN; WPABS; WPABSC; CNTXT; ENTXT; ENTXTC; CNKI; 万方数据知识服务平台, WANFANG; STN; Web of Science; Elsevier Science Direct; PubMed; 百度学术, BAIDU SCHOLAR: 己酸羟孕酮, 羟孕柄己酸酯, 羟孕酮己酸酯, 己酸孕酮, 长效黄体酮, 羟基孕酮己酸酯, 羟孕酮己酸盐, 己酸羟基黄体酮, Hydroxyprogesterone Caproate, HPC, OHPC, Hydroxyprogesterone hexanoate, Delalutin, Progesterone caproate, Depo-proluton, Hormofort, Neolutin, Primolut Depot, Proluton Depot, Syngynon, 630-56-8, 免疫检查点抑制剂, immune checkpoint inhibitor, 细胞程序性死亡受体1, PD-1, 程序性死亡配体1, PD-L1, 单克隆抗体, 单抗, monoclonal antibody, McAb, 癌, 瘤, cancer, tumor, tumour, neoplasm, carcinoma, 黑色素瘤, melanoma, 乳腺癌, breast cancer, breast carcinoma, mammary cancer, 4T1, 肿瘤浸润淋巴细胞, tumor infiltrating lymphocyte, TIL, CD8+T细胞, CD8-POSITIVE T CELL, T细胞, T cell, T lymphocyte, 肿瘤微环境, tumor microenvironment, TME

**C. DOCUMENTS CONSIDERED TO BE RELEVANT**

| Category* | Citation of document, with indication, where appropriate, of the relevant passages | Relevant to claim No. |
| --- | --- | --- |
| X | WO 2016100619 A2 (RGENIX, INC.) 23 June 2016 (2016-06-23) claims 1-15, and description, p. 1, line 36 to p. 2, line 1, p. 21, line 28 to p. 25, line 7, compounds 66 and 68, and p. 42, lines 7-16 | 1-27 |
| X | CN 1623550 A (ZHANG WEI et al.) 08 June 2005 (2005-06-08) claims 2-4, and description, pp. 4-5, and embodiment 8 | 1-2, 6-16, 21-27 |
| Y | WO 2017053823 A1 (PHARMACYCLICS LLC.) 30 March 2017 (2017-03-30) description, paragraphs 2-5 | 1-27 |
| Y | CN 108159056 A (SHANGHAI LIPU BIOMEDICINE TECH CO., LTD.) 15 June 2018 (2018-06-15) claims 1 and 7-8, and description, paragraphs 3-4 | 1-27 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **31 May 2022** | **14 June 2022** |

| Name and mailing address of the ISA/CN | Authorized officer |
| --- | --- |
| **China National Intellectual Property Administration (ISA/ CN)** **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/080275**

**C.      DOCUMENTS CONSIDERED TO BE RELEVANT**

| Category* | Citation of document, with indication, where appropriate, of the relevant passages | Relevant to claim No. |
|---|---|---|
| X | CN 108159056 A (SHANGHAI LIPU BIOMEDICINE TECH CO., LTD.) 15 June 2018 (2018-06-15)<br>        claims 1 and 7-8, and description, paragraphs 3-4 | 1-2, 6-16, 21-27 |
| X | 陆惠娟等 (LU, Huijuan et al.). "己酸孕酮、三苯氧胺及氨基导眠能对子宫内膜癌作用的研究 (Role of Hydroxyprogesterone Caproate, Tamoxifen and Aminoglutethemide in Endometrial Careinoma)"<br>现代妇产科进展 (Progress in Obstetrics and Gynecology),<br>Vol. 3, No. 3, 30 September 1994 (1994-09-30),<br>ISSN: 1004-7379,<br>        pp. 203-207 and 295-296 | 1-2, 6-16, 21-27 |
| X | 包胜英等 (BAO, Shengying et al.). "己酸孕酮对子宫内膜癌疗效相关因素的研究 (Non-official translation: Study on Factors Related to the Efficacy of Progesterone Caproate on Endometrial Carcinoma)"<br>现代妇产科进展 (Progress in Obstetrics and Gynecology), No. 2, 30 June 1998 (1998-06-30),<br>ISSN: 1004-7379,<br>        pp. 150-152 | 1-2, 6-16, 21-27 |
| A | 曹海敬等 (CAO, Haijing et al.). "子宫内膜癌治疗药物的研究进展 (Non-official translation: Research Progress of Drugs for Endometrial Cancer)"<br>中国药理学会第十一届全国基础与临床生殖药理学术研讨会暨生殖药理新技术与新方法培训班论文汇编 (Non-official translation: Compilation of Papers of the 11st National Symposium on Basic and Clinical Reproductive Pharmacology and Reproductive Pharmacology New Technology and Method Training Course of the Chinese Pharmacological Society), 20 September 2019 (2019-09-20),<br>        pp. 57-58 | 1-27 |
| A | ONSRUD, M. "Adjuvant hydroxyprogesterone caproate in stage I endometrial carcinoma1: changes in numbers and reactivities of some blood lymphocyte subpopulations."<br>Gynecologic Oncology, Vol. 14, No. 3, 31 December 1982 (1982-12-31),<br>ISSN: 0090-8258,<br>        pp. 355-364 | 1-27 |

Form PCT/ISA/210 (second sheet) (January 2015)

**INTERNATIONAL SEARCH REPORT**

International application No.

**PCT/CN2022/080275**

| Box No. II | Observations where certain claims were found unsearchable (Continuation of item 2 of first sheet) |

This international search report has not been established in respect of certain claims under Article 17(2)(a) for the following reasons:

1. ☑ Claims Nos.: **11-27**
   because they relate to subject matter not required to be searched by this Authority, namely:

   [1] Claims 11-27 set forth a method for increasing the number of tumor infiltrating lymphocytes (TIL) when needed, or a method for treating cancer of a subject in need, both relating to a method for treating diseases, and falling within the subject matter as defined in PCT Rule 39.1(iv) that does not warrant a search. It is expected that the subject matter may be amended by the applicant to a corresponding pharmaceutical use. The international search report is made on the basis of the subject matter after a reasonably expected amendment.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (January 2015)

**INTERNATIONAL SEARCH REPORT**
Information on patent family members

International application No.

**PCT/CN2022/080275**

| Patent document cited in search report | | | Publication date (day/month/year) | Patent family member(s) | | | Publication date (day/month/year) |
|---|---|---|---|---|---|---|---|
| WO | 2016100619 | A2 | 23 June 2016 | US | 2019125745 | A1 | 02 May 2019 |
| | | | | WO | 2016100619 | A3 | 23 June 2016 |
| CN | 1623550 | A | 08 June 2005 | None | | | |
| WO | 2017053823 | A1 | 30 March 2017 | None | | | |
| CN | 108159056 | A | 15 June 2018 | None | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110265778

**Non-patent literature cited in the description**

- **RANDOLPH J. NOLLE.** *Science,* 2020
- *CHEMICAL ABSTRACTS,* 630-56-8